# EUROPEAN PATENT APPLICATION

(11) **EP 4 467 540 A1**
(43) Date of publication of application: **27.11.2024**
(21) Application number: 22921756.7
(22) Date of filing: 28.12.2022
(51) Int. Cl.: C07D 471/04

(54) **METHOD FOR PREPARING FINERENONE AND INTERMEDIATE THEREOF**

(30) Priority: 19.01.2022 CN 202210063391
(71) Applicant: Aurisco Pharmaceutical co., Ltd., Zhejiang 317200 (CN)
(72) Inventor: XIONG, Zhigang, Taizhou, Zhejiang 317200 (CN); TANG, Jian, Taizhou, Zhejiang 317200 (CN); SUN, Yong, Taizhou, Zhejiang 317200 (CN); ZHANG, Chao, Taizhou, Zhejiang 317200 (CN); WEI, Jiadong, Taizhou, Zhejiang 317200 (CN); CHU, Dingjun, Taizhou, Zhejiang 317200 (CN)
(74) Representative: Petraz, Gilberto Luigi
(86) International application number: PCT/CN2022/142973
(87) International publication number: WO 2023/138336

(57) **Abstract**

Disclosed is a method for preparing a finerenone intermediate compound of formula (I) from a diastereomeric tartrate by means of racemic resolution. A compound of formula I having an ee value higher than 99.5% can be obtained. The preparation method provided has mild reaction conditions, simple post-treatment, and no special reagents required, and is suitable for industrial production.

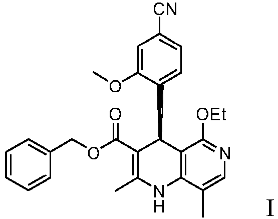

## Description

### FIELD OF THE INVENTION

The present invention belongs to the field of pharmaceutical chemistry technology, specifically relates to a method for preparing finerenone and the intermediates thereof.

### BACKGROUND FIELD

Finerenone is a nonsteroidal antagonist developed by Bayer Corporation to act as a mineralocorticoid receptor, and can be used as a drug for preventing and/or treating cardiovascular diseases and renal diseases such as heart failure and diabetes nephropathy. Its chemical name is (4*S*)-4-(4-cyano-2-methoxyphenyl)-5-ethoxy-2,8-dimethyl-1,4-dihydro-1,6-naphthalidine-3-formamide. The structural formula of finerenone is as follows:

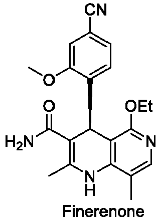

Currently, there are multiple literature reports the preparation methods of finerenone, among which the preparation method disclosed in WO2016016287 is shown in Route 1.

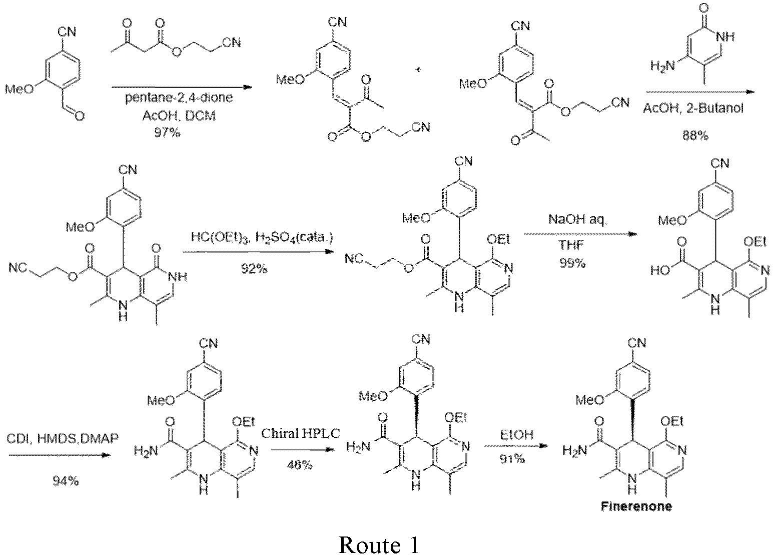

The preparation method disclosed in WO2017032673 is shown in Route 2.

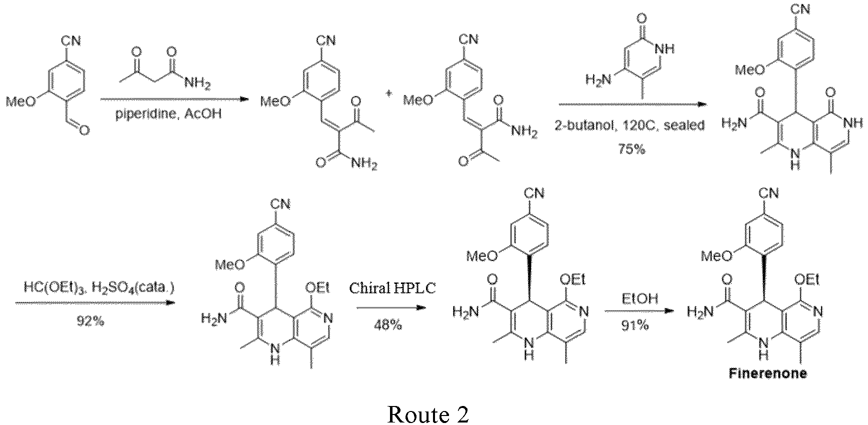

Both of the processes in Route 1 and Route 2 conduct resolution only after obtaining finerenone racemate, which is not economical in terms of overall yield. Moreover, they both use chiral HPLC resolution methods, which have very high costs of production and are not suitable for industrial large-scale production. In the later stage, WO2021074072 and WO2021074078 reported the processes wherein resolving agents was used to resolute intermediates, as shown in routes 3 and 4, respectively:

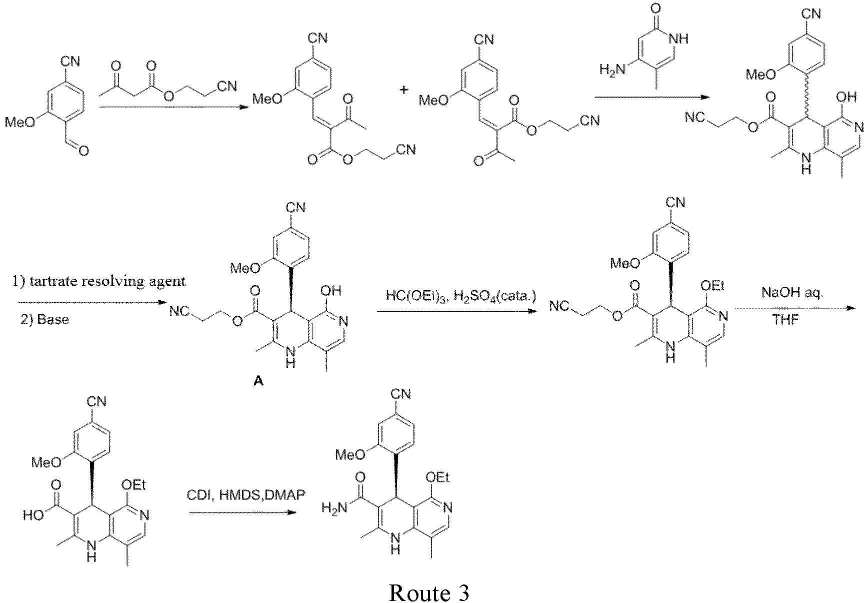

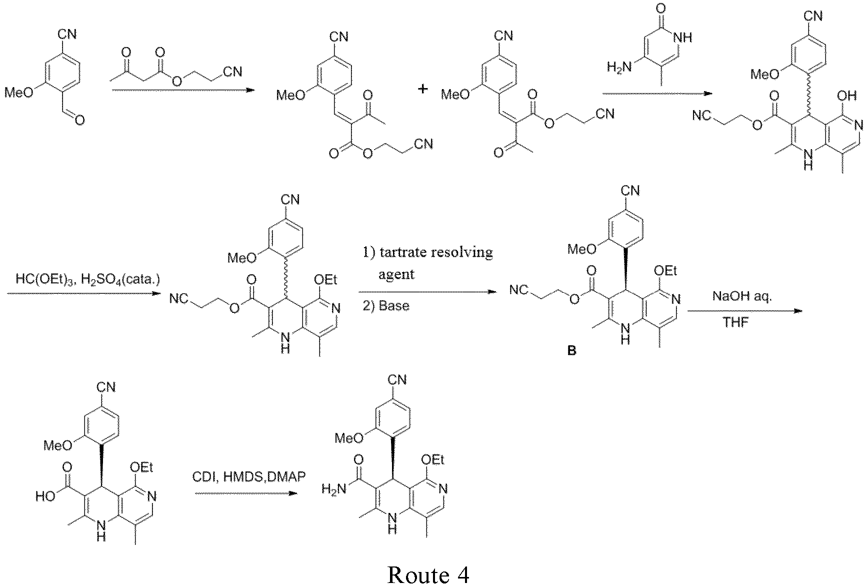

In Route 3, the diastereomeric salts intermediate yielded in resolution had a de value of less than 85%. After further purification, the de value of the diastereomeric salts increased to 98%. After making the salt free by adding alkali, the ee value of intermediate A was less than 99%, and the total resolution yield was less than 40%. In Route 4, the diastereomeric salts intermediate yielded in resolution had a de value of less than 80%. After further purification, the de value of the diastereomeric salts increased to 99%. After make the salt free by adding alkali, the ee value of intermediate B was 99%, and the total resolution yield was about 40%. The intermediates, diastereomeric salts, yielded in both routes had low de values and further purifications are required to increase ee values.

Therefore, there is an urgent need in this field to develop a process for preparing finerenone that is suitable for industrial production and has high yield, simple operation, better quality control.

### SUMMARY OF THE INVENTION

The technical problem to be solved by the present invention is to address the problems in the preparation of finerenone in the prior art such as high cost of racemic separation, low ee value of the product, and unsuitability for industrial production, thereby proposing a method for preparing finerenone.

The first aspect of the present invention provides a method for preparing a compound of formula I, comprising steps of:
(1) mixing a racemic compound of formula II with a resolving agent shown in compound of formula IIIa or formula IIIb, performing a salt formation reaction, and separating to obtain a salt shown in compound of formula IVa or formula IVc, respectively;
(2) treating the salt shown in the compound of formula IVa or formula IVc obtained in step 1 with alkali to obtain the compound of formula I;
   the reaction formula is as follows: 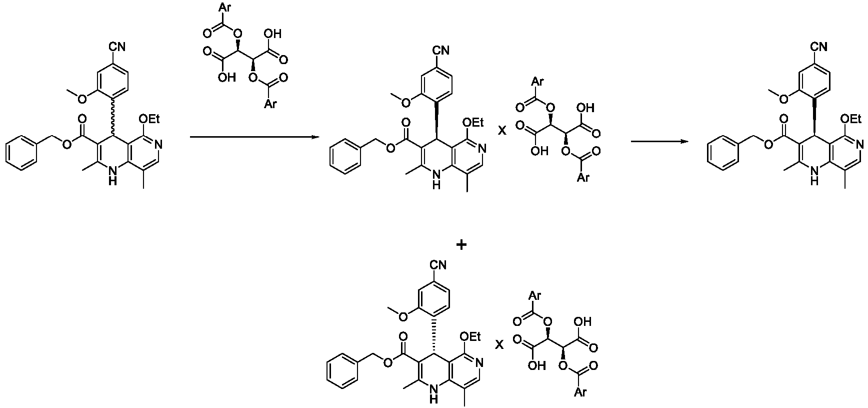 or 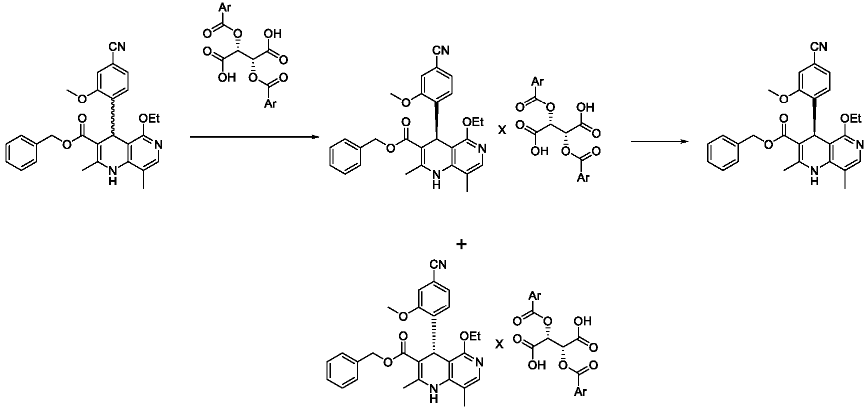 wherein, Ar is unsubstituted or substituted C6-C14 aromatic or heteroaromatic group, wherein the substituted refers to being substituted by the group selected from the group consisting of C1-C3 alkyl, C1-C3 haloalkyl, C1-C3 alkoxy, halogen, nitro, cyano, carboxyl, hydroxyl, and amido.

In another preferred embodiment, Ar is an unsubstituted or substituted phenyl.

In another preferred embodiment, Ar has a structure as shown in formula V: 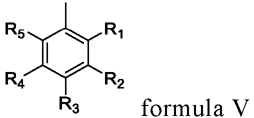 wherein, R₁, R₂, R₃, R₄, and R₅ are each independently hydrogen, halogen, C1-C3 alkyl, C1-C3 haloalkyl, C1-C3 alkoxy, phenoxy, nitro, cyano, or amido.

In another preferred embodiment, four substituents of R₁, R₂, R₃, R₄, and R₅ are hydrogen, and one substituent is not hydrogen, preferably R₃ is not hydrogen.

In another preferred embodiment, the amido refers to a group having a structure selected from the group consisting of -NHCOR, -NR'COR, -CONHR, and -CONRR', wherein each of R and R' are each independently methyl, ethyl, or phenyl, or N, R, R' and the carbon atoms to which they attached together form a 5-7 membered a heterocycle containing nitrogen.

In another preferred embodiment, Ar is an unsubstituted or substituted C10-C14 polycyclic aryl, such as naphthyl or anthryl.

In another preferred embodiment, Ar is an unsubstituted or substituted C5-C10 heteroaryl, such as a piperidinyl, piperazinyl, or quinolinyl.

In another preferred embodiment, Ar is selected from the group consisting of: 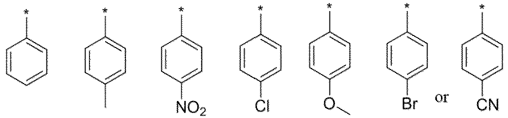 wherein, * represents the connection point.

In another preferred embodiment, Ar is a monosubstituted phenyl.

In another preferred embodiment, Ar is a para-substituted phenyl.

In another preferred embodiment, Ar is a phenmethyl, phenyl, nitrophenyl, chlorophenyl, bromophenyl, phenmethyloxy, or cyanophenyl.

In another preferred embodiment, Ar is a phenmethyl, phenyl, or phenoxy.

In another preferred embodiment, Ar is a phenyl or phenmethyl.

In another preferred embodiment, Ar is a phenmethyl.

In another preferred embodiment, in step 1, the molar ratio of compound of formula II to compound of formula IIIa is 1:0.4-1.2, preferably 1:0.6-1.2, and more preferably 1:1.

In another preferred embodiment, in step 1, the molar ratio of compound of formula II to compound of formula IIIb is 1:0.4-1.2, preferably 1:0.6-1.2, and more preferably 1:1.

In another preferred embodiment, in step 1, the resolving agent is a compound of formula IIIb.

In another preferred embodiment, in step 1, the salt formation reaction is carried out in organic solvent or a mixed solvent of water and organic solvent, wherein the organic solvent is selected from the group consisting of acetone, 2-butanone, methyl isobutyl ketone, ethyl acetate, dichloromethane, tetrahydrofuran, 2-methyltetrahydrofuran, ethylene glycol dimethyl ether or dioxane, and the combination thereof.

In another preferred embodiment, in step 1, the salt formation reaction further comprises one or more of the following features:
(a) the concentration of the compound of formula II in organic solvent is 0.1-0.3 mmol/mL, preferably 0.14-0.23 mmol/mL;
(b) the salt formation reaction is reacted at 60-70 °C;
(c) the reaction time for the salt formation reaction is 2-4 hours, preferably 3 hours.

In another preferred embodiment, the separation described in step 1 is precipitation separation.

In another preferred embodiment, in step 1, the separation comprises: cooling the reaction system to room temperature, stirring for 12-18 hours to precipitate, and separating by filteration.

In another preferred embodiment, the solid obtained by filtration separation is the compound of formula IVa or a compound of formula IVc, the compound of formula IVb or compound of formula IVd in the filtrate can be treated with alkali and then recovered as diastereoisomer using the method disclosed in WO2017032678.

In another preferred embodiment, in step 2, the alkali is potassium hydroxide, potassium phosphate, or sodium phosphate.

In another preferred embodiment, the alkali treatment step of step 2 is carried out in water, organic solvent, or a mixed solvent of water and organic solvent, wherein the organic solvent is selected from the group consisting of acetone, 2-butanone, methyl isobutyl ketone, ethyl acetate, dichloromethane, tetrahydrofuran, 2-methyltetrahydrofuran, ethylene glycol dimethyl ether or dioxane, and the combination thereof.

In another preferred embodiment, in step 2, the alkali treatment is carried out in an aqueous solution of alkali.

In another preferred embodiment, in step 2, the pH of the alkali treatment step is 7-7.5.

In another preferred embodiment, in step 2, the alkali treatment further comprises one or more of the following features:
(a) the concentration of the resolved salt shown in the compound of formula IVa or compound of formula IVc in the solvent are each independently 0.05-0.15 g/mL;
(b) the alkaline treatment is reacted at 15-25 °C;
(c) the reaction time for the alkali treatment is 2-4 hours, preferably 3 hours.

In another preferred embodiment, the compound of formula II is prepared by the following steps:
i) reacting compound 1 with 2-benzyl 3-oxobutyrate to obtain a mixture of compound 2 and compound 2';
ii) reacting the mixture obtained in step i with 4-amino-5-methylpyridinone to obtain compound 3;
iii) reacting compound 3 with triethyl orthoformate under the catalysis of concentrated sulfuric acid to obtain compound of formula II;
the reaction formula is as follows:

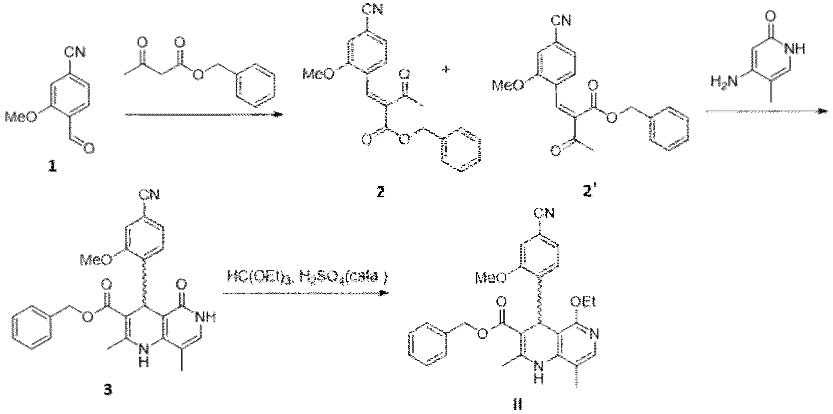

The second aspect of the present invention provides a method for preparing finerenone, wherein the method comprises the following steps:
(s1) providing a compound of formula II;
(s2) using the compound of formula II as raw material, subjecting it to resolution to prepare the compound of formula I, wherein the method for preparing the compound of formula I is as described in the first aspect of the present invention;
(s3) reducing the compound of formula I to obtain a compound of formula I-1;
(s4) ammoniating the compound of formula I-1 to obtain finerenone; the reaction formula is as follows:

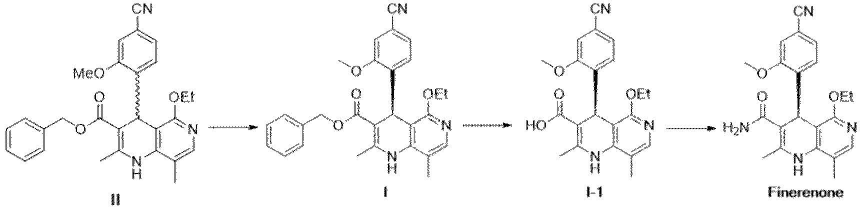

In another preferred embodiment, in step (s1), the compound of formula II is prepared by the following steps:
i) reacting compound 1 with 2-benzyl 3-oxobutyrate to obtain a mixture of compound 2 and compound 2';
ii) reacting the mixture obtained in step i with 4-amino-5-methylpyridinone to obtain compound 3;
iii) reacting compound 3 with triethyl orthoformate under the catalysis of concentrated sulfuric acid to obtain compound of formula II;
the reaction formula is as follows:

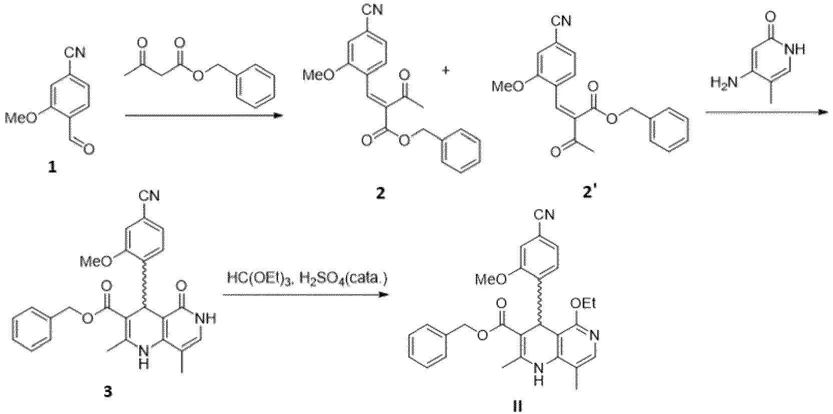

The third aspect of the present invention provides a diastereoisomeric salt or a pharmaceutically acceptable salt thereof as follows: 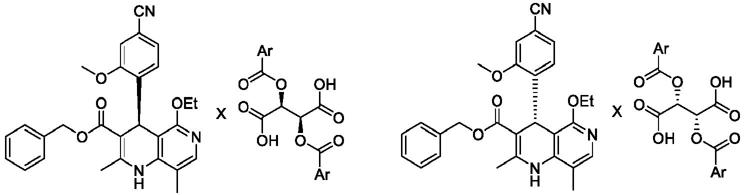 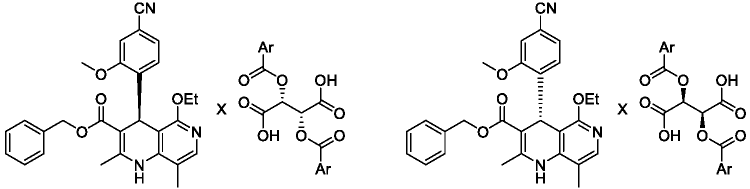 wherein, Ar is defined in the first aspect of the present invention.

In another preferred embodiment, the diastereoisomeric salt is IVa or IVc.

In another preferred embodiment, the diastereoisomeric salt is IVc.

In another preferred embodiment, the diastereoisomeric salt is any of diastereoisomeric salts selected from the group consisting of IVa-1, Iva-2, IVc-1 to IVc-6.

The fourth aspect of the present invention provides an intermediate of finerenone, the intermediate is a compound of formula II or an isomer or a pharmaceutically acceptable salt thereof

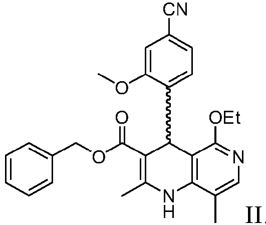

In another preferred embodiment, the intermediate is a racemic compound of formula II.

In another preferred embodiment, the intermediate is the (S)-isomer of the compound of formula II, i.e. the compound of formula I

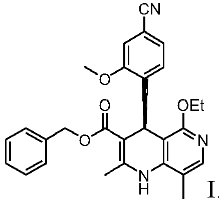

The fifth aspect of the present invention provides a use of the diastereoisomeric salt or the pharmaceutically acceptable salt thereof as described in the third aspect of the present invention, and the compound of formula II or an enantiomer or a pharmaceutically acceptable salt thereof as described in the fourth aspect of the present invention as an intermediate in the preparation process of finerenone for preparing finerenone.

### DETAILED DESCRIPTION OF THE INVENTION

After extensive and in-depth research, the inventor unexpectedly discovered a method for preparing finerenone with ultra-high ee value for the first time. Specifically, the inventor discovered that the use of a specific intermediate to be separated, after the cyanoethyl of the intermediate being replaced with phenmethyl, only simple separation and no further purification are required without to obtain diastereoisomeric salts with a de value exceeding 98.5%, then the intermediate obtained after alkali dissociation had an ee value of up to 99.8%, ultimately obtaining finerenone with an ee value exceeding 99.9%. This method is easy to operate, has a high yield, and a high product purity, making it very suitable for industrial production of finerenone. On this basis, the inventor completed the present invention.

### Intermediate of finerenone

The present invention provides an intermediate of finerenone, the intermediate is a racemic compound of formula II or a pharmaceutically acceptable salt thereof; 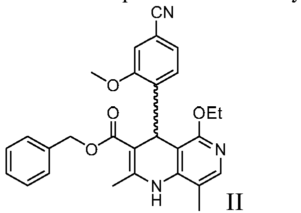 wherein, " 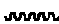 " represents the racemic structure.

The present invention also provides an intermediate of finerenone, the intermediate is a compound of formula I, i.e. the (S)-isomer obtained by resolving the compound of formula II.

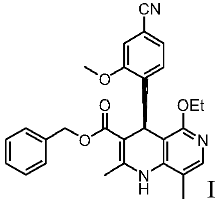

The carboxyl of the resolving agent in the present invention forms a diastereoisomeric resolved salt with the secondary amino group of the dihydropyridine ring in the compound of formula II. The dihydropyridine ring is a rigid planar structure, so the steric hindrance of the ester group connecting the chiral center should not have a significant impact on the resolution effect.

Surprisingly, after replacing the cyanoethyl of the compound to be separated with phenmethyl (compound of formula II) in the present invention, it was found that during the resolution reaction, the de value of the diastereoisomeric salt could reach 98.5% and more only via simple separation without further purification.

Method for preparing a finerenone intermediate compound of formula I

The present invention provides a method for preparing a finerenone intermediate compound of formula I, comprising steps of:
(1) reacting the racemic compound of formula II with the resolving agent shown in compound of formula IIIa or formula IIIb, separating to obtain the salt shown in compound IVa or compound IVc, respectively;
(2) subjecting the salt obtained in step 1 to alkalization and precipitation to obtain the compound of formula I.

The reaction formula is as follows: 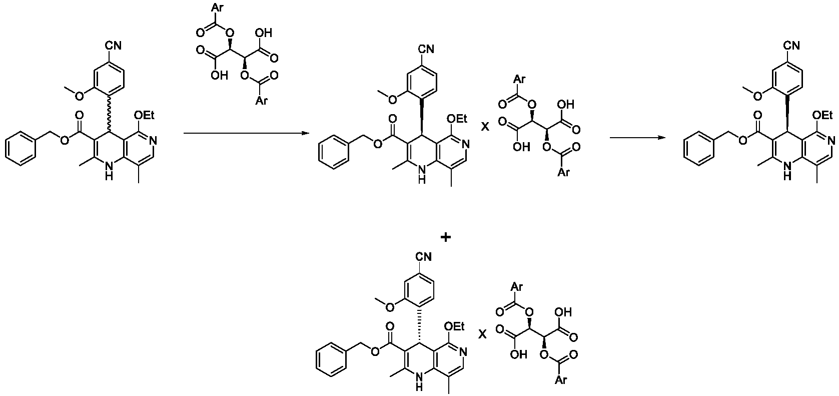 or 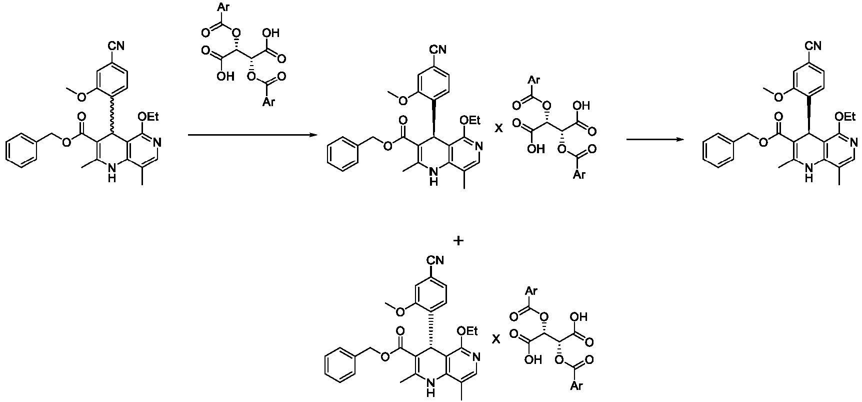 wherein, Ar represents unsubstituted or substituted aromatic or heteroaromatic group, and preferrably Ar represents: 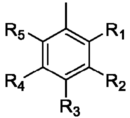
wherein, R₁, R₂, R₃, R₄, and R₅ each represents hydrogen atom or alkyl, such as methyl, ethyl, and propyl; or
halogen atoms, such as fluorine, chlorine, bromine, or iodine; or
ether group, such as O-methyl, O-ethyl, O-phenyl; or
nitro, cyano, CF₃, or
amide group, wherein the amide group is, for example, NHCOR, wherein R can represent methyl, ethyl, or phenyl, or -NRCOR group, wherein R has the above meaning, or CONHR group, wherein R has the above meaning, or CONRR' group, wherein R' has the same meaning as R defined above, or represents a cyclic amide such as -CO-piperidinyl. There may be significant differences in substitution methods.

Therefore, theoretically there may be up to 5 different substituents, but a monosubstituted Ar group is usually preferred.

However, Ar can also be a substituted heteroaromatic group, such as preferably piperidine or piperazine.

Ar can also be polycyclic aromatic hydrocarbon, such as substituted naphthalene, anthracene, or quinoline.

The specially preferred Ar is: 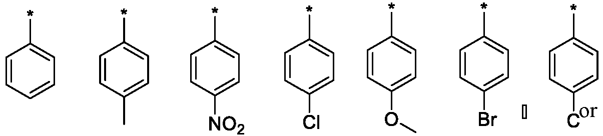 wherein * represents the connection point.

The more particularly preferred Ar is phenyl or methylbenzene.

In another preferred embodiment, in step 1, the molar ratio of compound of formula II to compound of formula IIIa or compound of formula IIIb is 1:0.4-1.2, preferably 1:0.6-1.2.

In another preferred embodiment, the salt formation reaction of step 1 is carried out in organic solvent or a mixed solvent of water and organic solvent, wherein the organic solvent is selected from the group consisting of acetone, 2-butanone, methyl isobutyl ketone, ethyl acetate, dichloromethane, tetrahydrofuran, dimethyltetrahydrofuran, ethylene glycol dimethyl ether or dioxane, and the combination thereof.

In another preferred embodiment, the separation in step 1 is precipitation separation.

The solid obtained by filtration is the compound of formula IVa or compound of formula IVc. The compound of formula IVb or compound of formula IVd in the filtrate can be treated with alkali and then recovered as diastereoisomer using the method disclosed in WO2017032678.

In another preferred embodiment, in step 2, the alkali is potassium hydroxide, potassium phosphate, or sodium phosphate.

In another preferred embodiment, the alkali treatment step of step 2 is carried out in water, organic solvent, or a mixed solvent of water and organic solvent, wherein the organic solvent is selected from the group consisting of acetone, 2-butanone, methyl isobutyl ketone, ethyl acetate, dichloromethane, tetrahydrofuran, dimethyltetrahydrofuran, ethylene glycol dimethyl ether or dioxane, and the combination thereof.

### A method for preparing finerenone

The present invention provides a method for preparing finerenone. The present invention uses a compound of formula II as the intermediate to be separated, from which a compound of formula I with high ee value can be obtained after simple separation to be used for preparing non noridone.

The method for preparing finerenone according to the present invention comprises the following steps:
(s1) providing a compound of formula II;
(s2) using the compound of formula II as raw material, subjecting to separation to prepare a compound of formula I, wherein the method for preparing compound of formula I is as described in the present invention;
(s3) reducing compound of formula I by palladium carbon to obtain a compound of formula 1-1;
(s4) ammoniating the compound of formula I-1 to obtain finerenone;
the reaction formula is as follows:

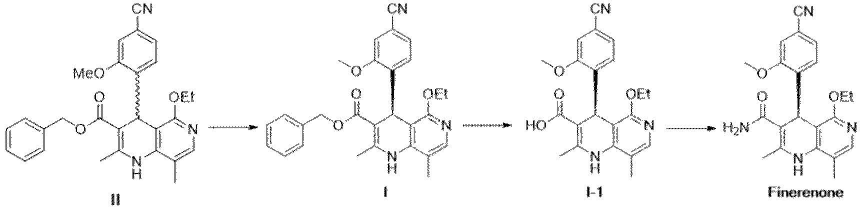

**The advantages of the preparation method of the present invention lie in that:**
(a) The reaction steps are simple, the reaction conditions are mild, no special reagents are required, the operation is simple, and it is suitable for industrial production.
(b) The separation of enantiomeric mixture using tartrates can avoid the use of costly and inefficient chiral chromatography methods. Moreover, the yield of enantiomeric mixture resolved by tartrate is over 65%, the de value of diastereoisomeric salt in intermediate is over 98.5%, the ee value of intermediate can reach 99.5% or more, and the ee value of the final product, finerenone, can reach 99.8% or more.

The present invention will be further explained below in combination with specific examples. It should be understood that these examples are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

Unless otherwise specified, the raw material or reagent used in the examples is commercially available or can be prepared using conventional methods.

The raw material 4-formyl-3-methoxybenzyronitrile used in the examples can be prepared by the method disclosed in WO2017032673.

### Example 1. Preparation of compound 3

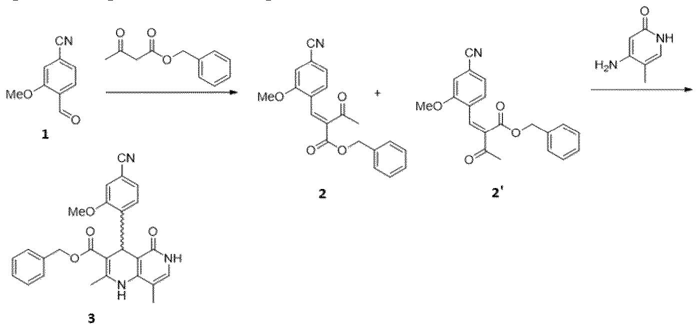

In a manifold, the mixture of 4-formyl-3-methoxybenzylnitrile (30.6g, 189.87mmol), 2-benzyl 3-oxobutyrate (45.6g, 237.34mmon), piperidine (1.9ml), and acetic acid (2.2ml) in dichloromethane (300ml) was reacted under reflux condition for 18 hours. The mixture was cooled to room temperature and the organic phase was washed twice with water each time. Then dichloromethane was concentrated to obtain a viscous liquid, the liquid was dissolved in 2-butanol (380ml), and 4-amino-5-methylpyridinone (20.8g, 167.13mmol) was added. The mixture was heated up to reflux and stirred for 20 hours. The mixture was cooled to 0 °C and stirred at this temperature for 4 hours, filtered, and rinsed with 2-butanol (100ml). The mixture was dried under vacuum at 40 °C for 16 hours to obtain 63.5g of light yellow solid with a yield of 86.0% (calculated based on 4-amino-5-methylpyridinone) and an HPLC purity of 99.5%.

### Example 2. Preparation of compound of formula II

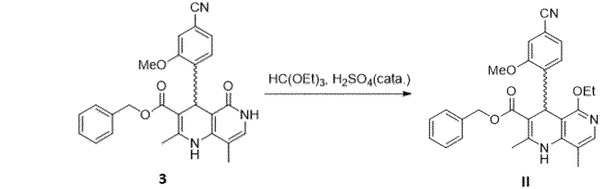

2-Benzyl 4-(4-cyano-2-methoxyphenyl) -2,8-dimethyl-5-oxo-1,4,5,6-tetrahydro-1,6- naphthyridine-3-formate (40.0g, 90.6mmol) and triethyl orthoformate (268.6g, 1812.1mmol) were dissolved in N,N-dimethylacetamide (1000ml), and concentrated sulfuric acid (1.2ml) was added. The mixture was heated at 115 °C for 2 hours, then cooled to room temperature. N,N-dimethylacetamide was concentrated under reduced pressure. Dichloromethane (1000ml) and water (1000ml) were added, the mixture was stirred and stood for layering. The organic phase was concentrated, methanol (500ml) was added, and the mixture was beaten to obtain 38.3g of light yellow solid with a yield of 90.1% and a HPLC purity of 99.8%. ¹H NMR(400 MHz, [D₆]DMSO): δ=1.05 (t, 3H), 2.12(s, 3H), 2.18 (s, 3H), 3.82 (s, 3H), 3.96 - 4.06 (m, 2H), 5.37 (s, 1H),6.49 - 6.86 (2H), 7.15 (d, 1H), 7.28 (d, 1H), 7.30 (m, 5H), 7.37 (d, 1H), 7.55 (s, 1H), 7.68 (s, 1H)_{∘} MS: m/z=470 [M+1]⁺.

### Example 3. Preparation of formula IVa-1

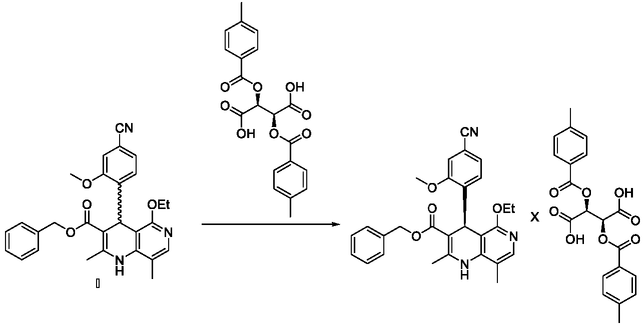

Compound of formula II (5.0g, 10.6mmol), compound of formula IIIa-1 (4.1g, 10.6mmol) (Ar in compound of formula IIIa is phenmethyl), and methyl isobutyl ketone (75ml) were added to a reaction flask at room temperature (about 20 °C), then the mixture was heated to an internal temperature of 70 °C for dissolution and stirred for 3 hours. Subsequently, the mixture was cooled to 20 °C within 5 hours, then stirred at this temperature for 15 hours, filtered, and the solid was rinsed with methyl isobutyl ketone (2.5ml), dried under vacuum at 45 °C to obtain 4.7g white solid (104.0% of the theoretical value) with a de value of 99.0%.

### Example 4. Preparation of formula IVa-2

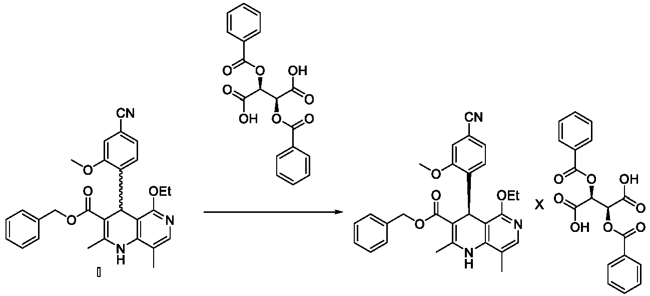

Compound of formula II (5.0g, 10.6mmol), compound of formula IIIa-2 (3.8g, 10.6mmol) (Ar in compound of formula IIIa is phenyl), and ethylene glycol dimethyl ether (75ml) were added to a reaction flask at room temperature (about 20 °C), then the mixture was heated to an internal temperature of 70 °C for dissolution and stirred for 3 hours. Subsequently, the mixture was cooled to 20 °C within 5 hours, then stirred at this temperature for 15 hours, filtered, and the solid was rinsed with ethylene glycol dimethyl ether (2.5ml), dried under vacuum at 45 °C to obtain 4.3g white solid (98.0% of the theoretical value) with a de value of 99.2%.

### Example 5. Preparation of formula IVc-1

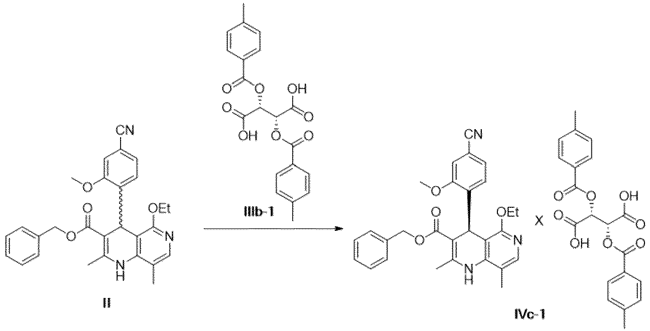

Compound of formula II (5.0g, 10.6mmol), compound of formula IIIb-1 (4.1g, 10.6mmol) (Ar in compound of formula IIIb is phenmethyl), and tetrahydrofuran (75ml) were added to a reaction flask at room temperature (about 20 °C), then the mixture was heated to an internal temperature of 60 °C for dissolution and stirred for 3 hours. Subsequently, the mixture was cooled to 20 °C within 5 hours, then stirred at this temperature for 15 hours, filtered, and the solid was rinsed with tetrahydrofuran (2.5ml), dried under vacuum at 45 °C to obtain 4.7g white solid (104.0% of the theoretical value) with a de value of 99.1%.

### Example 6. Preparation of formula IVc-2

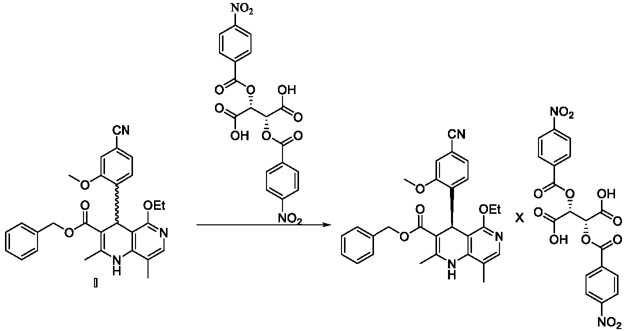

Compound of formula II (5.0g, 10.6mmol), compound of formula IIIb-2 (4.8g, 10.6mmol) (Ar in compound of formula IIIb is nitrophenyl), and tetrahydrofuran (50ml) were added to a reaction flask at room temperature (about 20 °C), then the mixture was heated to an internal temperature of 60 °C for dissolution and stirred for 3 hours. Subsequently, the mixture was cooled to 20 °C within 5 hours, then stirred at this temperature for 15 hours, filtered, and the solid was rinsed with tetrahydrofuran (2.5ml), dried under vacuum at 45 °C to obtain 4.9g white solid (100.0% of the theoretical value) with a de value of 98.8%.

### Example 7. Preparation of formula IVc-3

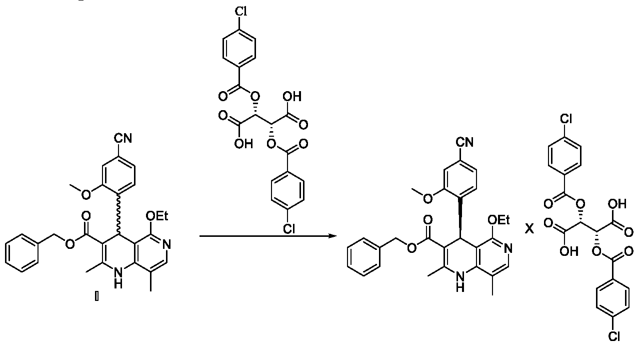

Compound of formula II (5.0g, 10.6mmol), compound of formula IIIb-3 (4.5g, 10.6mmol) (Ar in compound of formula IIIb is chlorophenyl), and acetone (45ml) were added to a reaction flask at room temperature (about 20 °C), then the mixture was heated to an internal temperature of 60 °C for dissolution and stirred for 3 hours. Subsequently, the mixture was cooled to 20 °C within 5 hours, then stirred at this temperature for 15 hours, filtered, and the solid was rinsed with acetone (2.5ml), dried under vacuum at 45 °C to obtain 4.8g white solid (100.0% of the theoretical value) with a de value of 99.0%.

### Example 8. Preparation of formula IVc-4

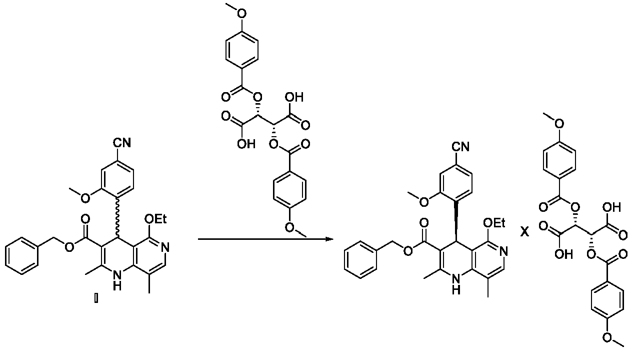

Compound of formula II (5.0g, 10.6mmol), compound of formula IIIb-4 (2.7g, 6.4mmol) (Ar in compound of formula IIIb is phenylmethoxy), and ethyl acetate (75ml) were added to a reaction flask at room temperature (about 20 °C), then the mixture was heated to an internal temperature of 60 °C for dissolution and stirred for 3 hours. Subsequently, the mixture was cooled to 20 °C within 5 hours, then stirred at this temperature for 15 hours, filtered, and the solid was rinsed with ethyl acetate (2.5ml), dried under vacuum at 45 °C to obtain 4.7g white solid (100.0% of the theoretical value) with a de value of 99.2%.

### Example 9. Preparation of formula IVc-5

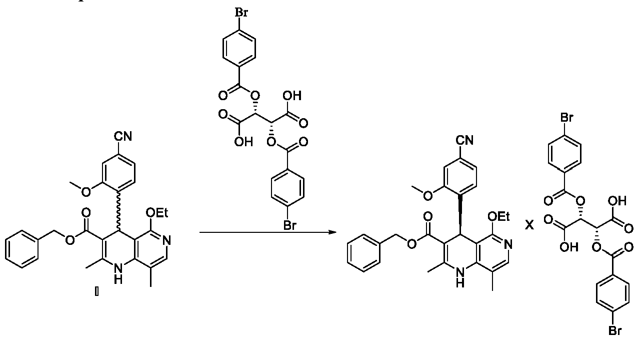

Compound of formula II (5.0g, 10.6mmol), compound of formula IIIb-5 (6.6g, 12.7mmol) (Ar in compound of formula IIIb is bromophenyl), and dichloromethane (75ml) were added to a reaction flask at room temperature (about 20 °C), then the mixture was heated to an internal temperature of 35 °C for dissolution and stirred for 3 hours. Subsequently, the mixture was cooled to 20 °C within 5 hours, then stirred at this temperature for 15 hours, filtered, and the solid was rinsed with dichloromethane (2.5ml), dried under vacuum at 45 °C to obtain 5.0g white solid (95.0% of the theoretical value) with a de value of 99.2%.

### Example 10. Preparation of formula IVc-6

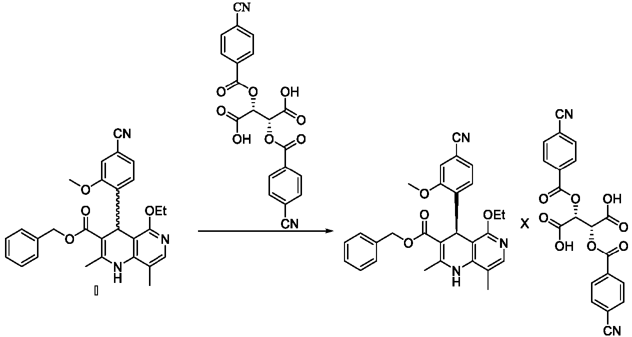

Compound of formula II (5.0g, 10.6mmol), compound of formula IIIb-6 (4.3g, 10.6 mmol) (Ar in compound of formula IIIb is cyanophenyl), and tetrahydrofuran (75ml) were added to a reaction flask at room temperature (about 20 °C), then the mixture was heated to an internal temperature of 60 °C for dissolution and stirred for 3 hours. Subsequently, the mixture was cooled to 20 °C within 5 hours, then stirred at this temperature for 15 hours, filtered, and the solid was rinsed with tetrahydrofuran (2.5ml), dried under vacuum at 45 °C to obtain 4.6g white solid (98.9% of the theoretical value) with a de value of 99.4%.

### Example 11. Preparation of formula IVc-1

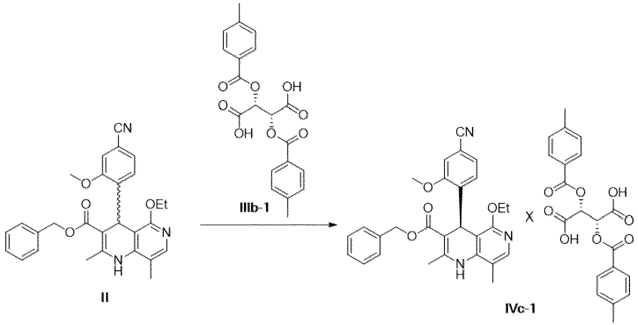

Compound of formula II (5.0g, 10.6mmol), compound of formula IIIb-1 (4.1g, 10.6 mmol) (Ar in compound of formula IIIb is phenmethyl), and 2-methyltetrahydrofuran (75ml) were added to a reaction flask at room temperature (about 20 °C), then the mixture was heated to an internal temperature of 60 °C for dissolution and stirred for 3 hours. Subsequently, the mixture was cooled to 20 °C within 5 hours, then stirred at this temperature for 15 hours, filtered, and the solid was rinsed with 2-methyltetrahydrofuran (2.5ml), dried under vacuum at 45 °C to obtain 4.6g white solid (101.0% of the theoretical value) with a de value of 99.5%.

### Example 12. Preparation of formula IVc-1

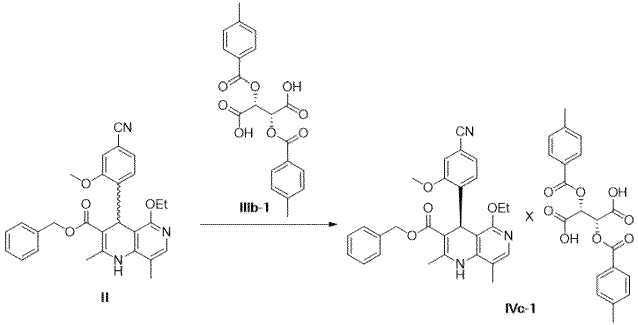

Compound of formula II (5.0g, 10.6mmol), compound of formula IIIb-1 (4.1g, 10.6 mmol) (Ar in compound of formula IIIb is phenmethyl), and ethylene glycol dimethyl ether (100ml) were added to a reaction flask at room temperature (about 20 °C), then the mixture was heated to an internal temperature of 70 °C for dissolution and stirred for 3 hours. Subsequently, the mixture was cooled to 20 °C within 5 hours, then stirred at this temperature for 15 hours, filtered, and the solid was rinsed with ethylene glycol dimethyl ether (2.5ml), dried under vacuum at 45 °C to obtain 4.5g white solid (98.9% of the theoretical value) with a de value of 99.4%.

### Example 13. Preparation of formula IVc-1

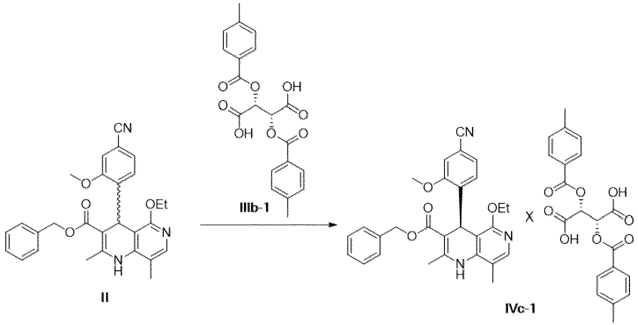

Compound of formula II (5.0g, 10.6mmol), compound of formula IIIb-1 (4.1g, 10.6 mmol) (Ar in compound of formula IIIb is phenmethyl), and dioxane (75ml) were added to a reaction flask at room temperature (about 20 °C), then the mixture was heated to an internal temperature of 70 °C for dissolution and stirred for 3 hours. Subsequently, the mixture was cooled to 20 °C within 5 hours, then stirred at this temperature for 15 hours, filtered, and the solid was rinsed with dioxane (2.5ml), dried under vacuum at 45 °C to obtain 4.5g white solid (98.9% of the theoretical value) with a de value of 99.6%.

### Example 14. Preparation of formula IVc-1

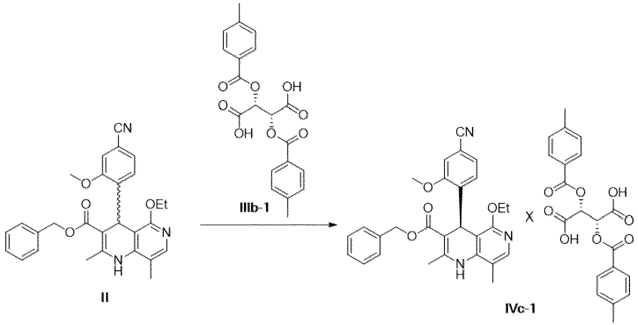

Compound of formula II (5.0g, 10.6mmol), compound of formula IIIb-1 (4.1g, 10.6 mmol) (Ar in compound of formula IIIb is phenmethyl), and 2-butanone (75ml) were added to a reaction flask at room temperature (about 20 °C), then the mixture was heated to an internal temperature of 70 °C for dissolution and stirred for 3 hours. Subsequently, the mixture was cooled to 20 °C within 5 hours, then stirred at this temperature for 15 hours, filtered, and the solid was rinsed with 2-butanone (2.5ml), dried under vacuum at 45 °C to obtain a white solid of 4.5g (98.9% of the theoretical value) with a de value of 99.0%.

### Example 15. Preparation of compound of formula I

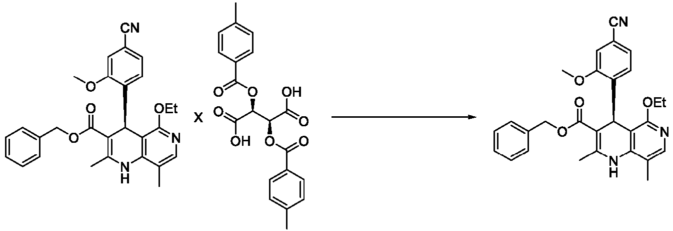

The resolved salt of formula IVa-1 (5.0g) and water (50ml) were added into a reaction flask at room temperature (about 20 °C), the mixture was stirred for 30 minutes, then sodium phosphate aqueous solution (100g sodium phosphate dissolved in 1000 ml water) was added dropwise to adjust the pH to 7-7.5, and the mixture was continued to stir for 3h. The mixture was filtered, the solid was rinsed with 20ml of water, dried under vacuum at 45°C to obtain 2.7g of white solid with a yield of 98.8%, HPLC purity of 99.7%, and ee value of 99.6%. MS: m/z=470 [M+1]⁺.

### Example 16. Preparation of compound of formula I

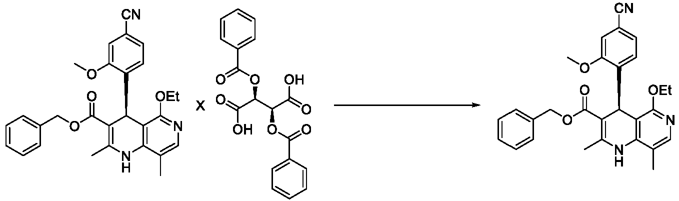

The resolved salt of formula IVa-2 (5.0g) and water (50ml) were added into a reaction flask at room temperature (about 20 °C), the mixture was stirred for 30 minutes, then sodium phosphate aqueous solution (100g sodium phosphate dissolved in 1000 ml water) was added dropwise to adjust the pH to 7-7.5, and the mixture was continued to stir for 3h. The mixture was filtered, the solid was rinsed with 20ml of water, dried under vacuum at 45°C to obtain 2.7g of white solid with a yield of 95.1%, HPLC purity of 99.8%, and ee value of 99.5%. MS: m/z=470 [M+1]⁺.

### Example 17. Preparation of compound of formula I

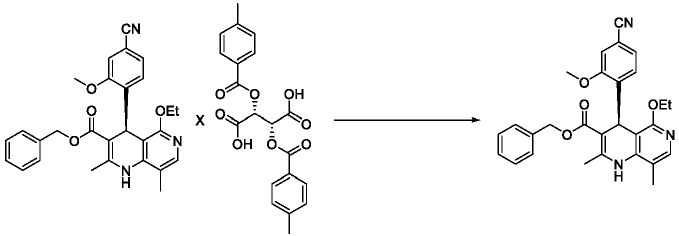

The resolved salt of formula IVc-1 (5.0g) and water (50ml) were added into a reaction flask at room temperature (about 20 °C), the mixture was stirred for 30 minutes, then sodium phosphate aqueous solution (100g sodium phosphate dissolved in 1000 ml water) was added drowise to adjust the pH to 7-7.5, and the mixture was continued to stir for 3h. The mixture was filtered, the solid was rinsed with 20ml of water, dried under vacuum at 45°C to obtain 2.6g of white solid with a yield of 94.9%, HPLC purity of 99.6%, and ee value of 98.6%. MS: m/z=470 [M+1]⁺.

### Example 18. Preparation of compound of formula I

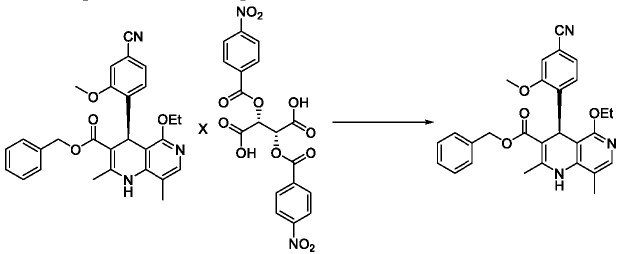

The resolved salt of formula IVc-2 (5.0g) and water (50ml) were added into a reaction flask at room temperature (about 20 °C), the mixture was stirred for 30 minutes, then sodium phosphate aqueous solution (100g sodium phosphate dissolved in 1000 ml water) was added dropwise to adjust the pH to 7-7.5, and the mixture was continued to stir for 3h. The mixture was filtered, the solid was rinsed with 20ml of water, dried under vacuum at 45°C to obtain 2.5g of white solid with a yield of 97.7%, HPLC purity of 99.7%, and ee value of 99.7%. MS: m/z=470 [M+1]⁺.

### Example 19. Preparation of compound of formula I

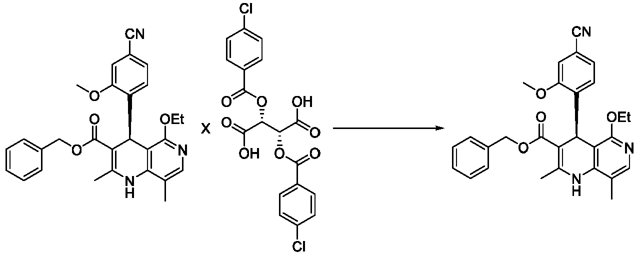

The resolved salt of formula IVc-3 (5.0g) and water (50ml) were added into a reaction flask at room temperature (about 20 °C), the mixture was stirred for 30 minutes, then sodium phosphate aqueous solution (100g sodium phosphate dissolved in 1000 ml water) was added dropwise to adjust the pH to 7-7.5, and the mixture was continued to stir for 3h. The mixture was filtered, the solid was rinsed with 20ml of water, dried under vacuum at 45°C to obtain 2.5g of white solid with a yield of 95.4%, HPLC purity of 99.5%, and ee value of 99.8%. MS: m/z=470 [M+1]⁺.

### Example 20. Preparation of compound of formula I

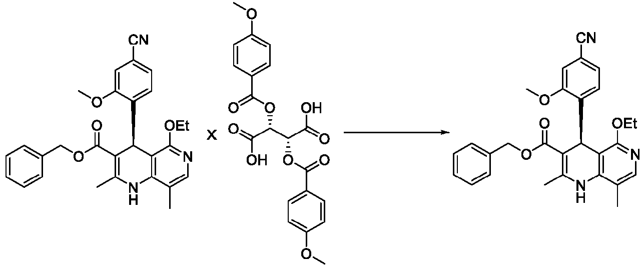

The resolved salt of formula IVc-4 (5.0g) and water (50ml) were added into a reaction flask at room temperature (about 20 °C), the mixture was stirred for 30 minutes, then sodium phosphate aqueous solution (100g sodium phosphate dissolved in 1000 ml water) was added dropwise to adjust the pH to 7-7.5, and the mixture was continued to stir for 3h. The mixture was filtered, the solid was rinsed with 20ml of water, dried under vacuum at 45°C to obtain 2.6g of white solid with a yield of 98.5%, HPLC purity of 99.5%, and ee value of 99.6%. MS: m/z=470 [M+1]⁺.

### Example 21. Preparation of compound of formula I

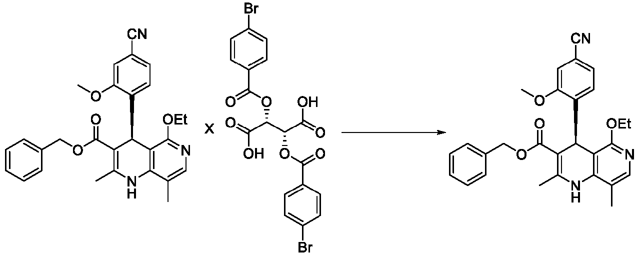

The resolved salt of formula IVc-5 (5.0g) and water (50ml) were added into a reaction flask at room temperature (about 20 °C), the mixture was stirred for 30 minutes, then sodium phosphate aqueous solution (100g sodium phosphate dissolved in 1000ml water) was added dropwise to adjust the pH to 7-7.5, and the mixture was continued to stir for 3h. The mixture was filtered, the solid was rinsed with 20ml of water, dried under vacuum at 45°C to obtain 2.3g of white solid with a yield of 96.6%, HPLC purity of 99.6%, and ee value of 99.5%. MS: m/z=470 [M+1]⁺.

### Example 22. Preparation of compound of formula I

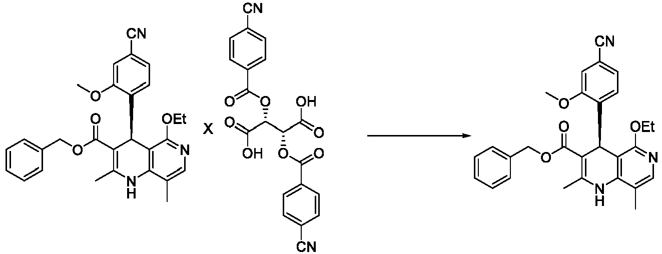

The resolved salt of formula IVc-6 (5.0g) and water (50ml) were added into a reaction flask at room temperature (about 20 °C), the mixture was stirred for 30 minutes, then sodium phosphate aqueous solution (100g sodium phosphate dissolved in 1000ml water) was added dropwise to adjust the pH to 7-7.5, and the mixture was continued to stir for 3h. The mixture was filtered, the solid was rinsed with 20ml of water, dried under vacuum at 45°C to obtain 2.5g of white solid with a yield of 93.6%, HPLC purity of 99.7%, and ee value of 99.7%. MS: m/z=470 [M+1]⁺.

### Example 23. Preparation of compound of formula I-1

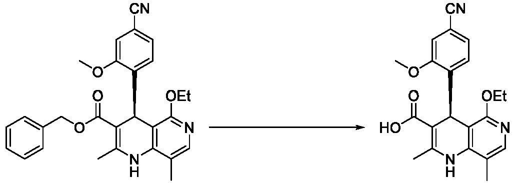

At room temperature (about 20 °C), to a reaction flask was added with compound of formula I (5.0g, 10.6mmol), tetrahydrofuran (50ml), 10% palladium carbon (0.5g) and replaced with nitrogen three times followed by hydrogen three times. The mixture was continued to stir at room temperature for 3 hours, palladium carbon was filtered, and the organic phase was concentrated to obtain 3.9g white solid with a yield of 98.0% and an ee value of 99.9%. HPLC purity is 99.7%. MS: m/z=380 [M+1]⁺.

### Example 24. Preparation of finerenone

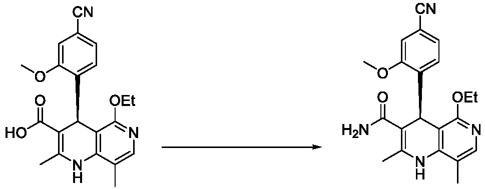

Compound of formula I-1 (1.6g, 4.22mmol), 1,1-carbonyldiimidazole (1.0g, 5.91mmol), tetrahydrofuran (8ml) were added to a reaction flask at room temperature (approximately 20 °C), and 4-dimethylaminopyridine (51mg, 0.42mmol) was added at room temperature. The mixture was stirred at room temperature for 1 hour, then heated to 50 °C and continued stirring for 2.5 hours. Hexamethyldisilazane (3.0g, 18.42mmol) was added to the solution and the mixed solution was stirred under reflux for 22 hours. Then, tetrahydrofuran (1.8ml) was added and the mixture was cooled to 5 °C. A mixture of 1.2ml tetrahydrofuran and 1.0ml water was added within 3 hours while control the internal temperature between 5 and 20 °C. Subsequently, the mixture was stirred under reflux for 1 hour, slowly cooled to 0 °C, and stirred at this temperature for 1 hour, filtered, and rinsed twice with 3ml of water. The mixture was dried under vacuum at 55°C to obtain 1.5g of a white solid with a yield of 94.0%, HPLC purity of 99.9%, and ee value of 100.0%. ¹H NMR (400 MHz, [D₆]DMSO): δ =1.05 (t, 3H), 2.12(s, 3H), 2.18 (s, 3H), 3.82 (s, 3H), 3.96 - 4.06 (m, 2H), 5.37 (s, 1H),6.49 - 6.86 (2H), 7.15 (d, 1H), 7.28 (d, 1H), 7.37 (d, 1H), 7.55 (s, 1H), 7.68 (s, 1H)_{∘} MS: m/z=379 [M+1]⁺.

## Claims

1. A method for preparing a compound of formula I, comprising steps of:
(1) mixing a racemic compound of formula II with a resolving agent shown in compound of formula IIIa or formula IIIb, performing a salt formation reaction, and separating to obtain a salt shown in compound of formula IVa or formula IVc, respectively;
(2) treating the salt shown in the compound of formula IVa or formula IVc obtained in step 1 with alkali to obtain the compound of formula I;
the reaction formula is as follows: 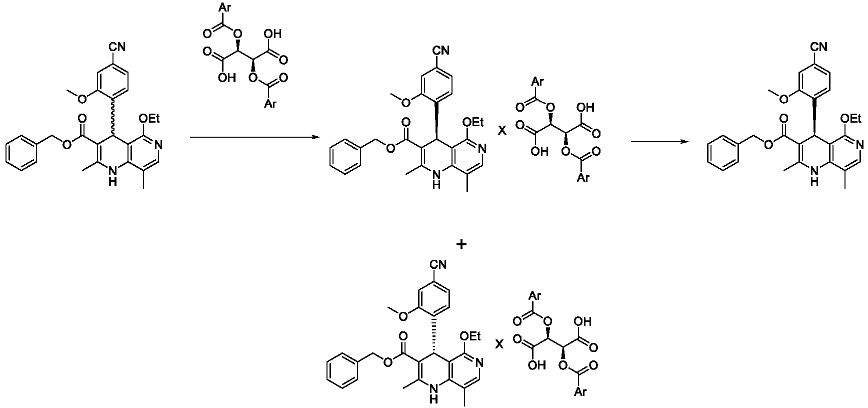 or 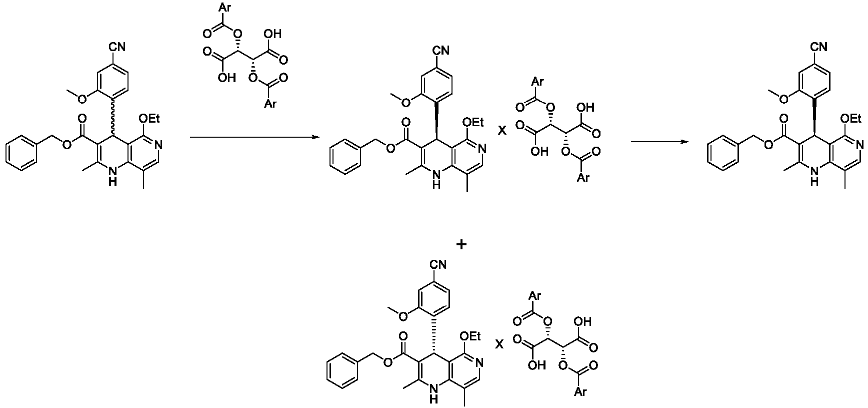 wherein, Ar is unsubstituted or substituted C6-C14 aromatic or heteroaromatic group, wherein the substituted refers to being substituted by the group selected from the group consisting of C1-C3 alkyl, C1-C3 haloalkyl, C1-C3 alkoxy, halogen, nitro, cyano, carboxyl, hydroxyl, and amido.

2. The method of claim 1, wherein Ar has a structure as shown in formula V: 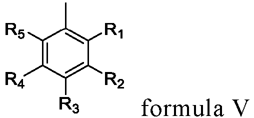 wherein, R₁, R₂, R₃, R₄, and R₅ are each independently hydrogen, halogen, C1-C3 alkyl, C1-C3 haloalkyl, C1-C3 alkoxy, phenoxy, nitro, cyano, or amido.

3. The method of claim 1, wherein Ar is selected from the group consisting of: 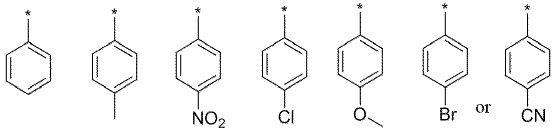 wherein, * represents the connection point.

4. The method of claim 1, wherein in step 1, the molar ratio of compound of formula II to compound of formula IIIa or compound of formula IIIb is 1: 0.4-1.2, preferably 1: 0.6-1.2, and more preferably 1:1.

5. The method of claim 1, wherein in step 1, the salt formation reaction is carried out in an organic solvent or a mixed solvent of water and organic solvent, wherein the organic solvent is selected from the group consisting of acetone, 2-butanone, methyl isobutyl ketone, ethyl acetate, dichloromethane, tetrahydrofuran, dimethyltetrahydrofuran, ethylene glycol dimethyl ether or dioxane, and the combination thereof.

6. The method of claim 1, wherein in step 2, the alkali is potassium hydroxide, potassium phosphate, or sodium phosphate.

7. The method of claim 1, wherein the compound of formula II is prepared by the following steps:
i) reacting compound 1 with 2-benzyl 3-oxobutyrate to obtain a mixture of compound 2 and compound 2';
ii) reacting the mixture obtained in step i with 4-amino-5-methylpyridinone to obtain compound 3;
iii) reacting compound 3 with triethyl orthoformate under the catalysis of concentrated sulfuric acid to obtain the compound of formula II; the reaction formula is as follows:

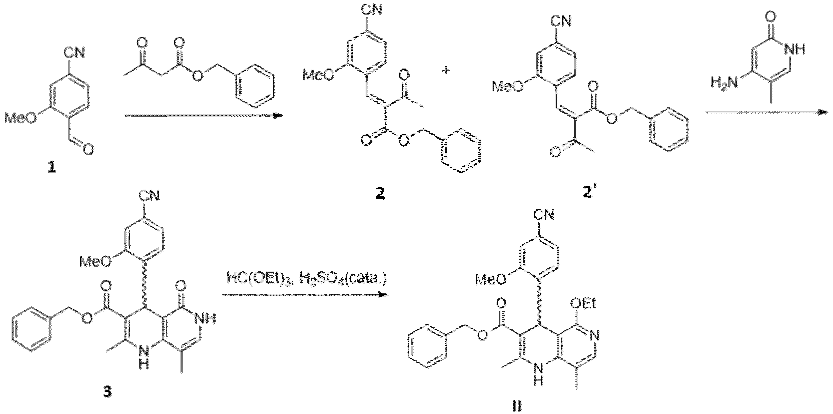

8. A method for preparing finerenone, wherein the method comprises the following steps:
(s1) providing a compound of formula II;
(s2) using the compound of formula II as raw material, subjecting to resolution to prepare a compound of formula I, wherein the method for preparing compound of formula I is as described in claim 1;
(s3) reducing the compound of formula I to obtain a compound of formula I-1;
(s4) ammoniating the compound of formula I-1 to obtain finerenone;
the reaction formula is as follows:

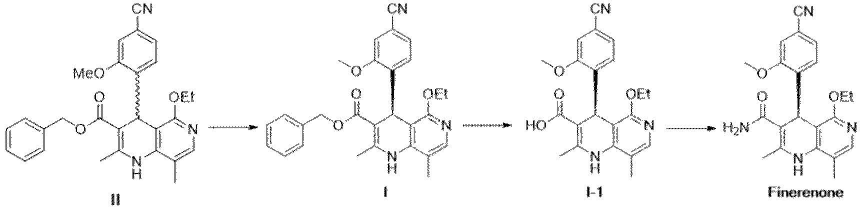

9. A diastereoisomeric salt or a pharmaceutically acceptable salt thereof as shown in following formulas: 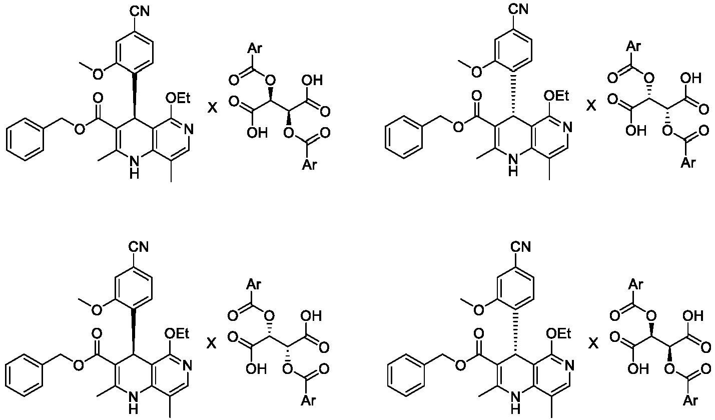 wherein, Ar is defined in any one of claims 1 to 3.

10. An intermediate of finerenone, wherein the intermediate is a compound of formula II or an enantiomer or a pharmaceutically acceptable salt thereof
